(19) Europäisches Patentamt
European Patent Office
Office européen des brevets

(11) Veröffentlichungsnummer: **0 001 225**
**B1**

(12) # EUROPÄISCHE PATENTSCHRIFT

(21) Anmeldenummer: **78100764.6**

(22) Anmeldetag: **28.08.78**

(51) Int. Cl³: **C 07 C 127/19**, A 01 N 47/30

(54) **Phenylharnstoffderivate, diese Verbindungen enthaltende Herbizide und Verwendung dieser Herbizide**

(30) Priorität: **01.09.77 DE 2739349**

(43) Veröffentlichungstag der Anmeldung:
**04.04.79 Patentblatt 79/07**

(45) Bekanntmachung des Hinweises auf die Patenterteilung:
**30.04.80 Patentblatt 80/09**

(84) Benannte Vertragsstaaten:
**BE CH DE FR GB LU NL SE**

(56) Entgegenhaltungen:
**CH - A - 537 148**
**DE - A - 2 436 108**
**DE - B - 1 062 059**
**FR - A - 2 037 825**
**GB - A - 951 423**
**GB - A - 1 255 258**
**US - A - 3 288 586**

(73) Patentinhaber: **BASF Aktiengesellschaft**
**Carl-Bosch-Strasse 38**
**D - 6700 Ludwigshafen (DE)**

(72) Erfinder: **Linhart, Friedrich, Dr.**
**Leisberg 61**
**D - 6900 Heidelberg (DE)**
**Zeeh, Bernd, Dr.**
**Thorwaldsenstrasse 5**
**D - 6700 Ludwigshafen (DE)**
**Jacobs, Peter, Dr.**
**Hanns-Fay-Strasse 4**
**D - 6710 Frankenthal (DE)**
**Wuerzer, Bruno, Dr.**
**Wilhelm-Busch-Strasse 55**
**D - 6703 (DE)**

**EP 0 001 225 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Phenylharnstoffderivate, diese Verbindungen enthaltende Herbizide und Verwendung dieser Herbizide.

Die vorliegende Erfindung betrifft neue wertvolle N-Phenyl-N'-methyl-N'-methoxyharnstoffderivate mit guter herbizider Wirkung und Herbizide, die diese Verbindungen als Wirkstoffe enthalten.

Es ist bekannt, daß N-(4-Isopropyl-phenyl)-N',N'-dimethyl-harnstoff (US—PS 2 655 447, DT—OS 2 107 774, DT—OS 2 039 041) eine gute Verträglichkeit für Winterweizen hat. Gleichzeitig beseitigt diese Verbindung unerwünschte Gräser wie Alopecurus myosuroides und einige breitblättrige Arten, Völlig unzulänglich bleibt die Wirkung gegen Galium aparine und andere dikotyle Species.

Betrachtet man parallel dazu den entsprechenden bekannten N-(4-Isopropyl-phenyl)-N'-methoxy-N'-methyl-harnstoff (DT—PS 1 062 059), so zeigt dieser eine ähnliche herbizide Aktivität.

Der bekannte N-(3-tert.-Butyl-phenyl)-N',N'-dimethylharnstoff (DT—OS 2 436 108) ist eine Verbindung mit intensiver herbizider Wirkung. Eine ausreichende Verträglichkeit für Getreide ist jedoch nicht mehr vorhanden. Einen hierzu im Durchschnitt etwas schwächeren Herbizideffekt bei gleichzeitig etwas günstigerer, wenngleich trotzdem noch sehr marginaler Getreideselektivität zeigt der bekannte N-(3-Methyl-phenyl)-N'-methoxy-N'-methylharnstoff (DT—OS 1 905 598).

Überraschend wurde gefunden, daß Methoxymethylharnstoffderivate der allgemeinen Formel

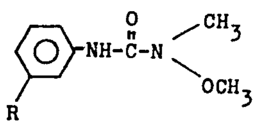,

in der R einen verzweigten Alkylrest mit 3 bis 5 C-Atomen (z.B. Isopropyl, Isobutyl, sek.-Butyl, tert.-Butyl, 1,1-Dimethylpropyl, 1-Äthylpropyl, 1-Methylbutyl, 2,2-Dimethylpropyl, 2-Methylbutyl oder 3-Methylbutyl) bedeutet, eine sehr gute herbizide Wirkung gegen unerwünschte monokotyle und dikotyle (grasartige und breitblättrige) Pflanzen, einschließlich Galium aparine, haben und gleichzeitig ein hohes Maß an Verträglichkeit für Getreide, insbesondere Wintergetriede (Roggen, Weizen, Gerste) bei Vor- und Nachauflaufanwendung besitzen.

Die erfindungsgemäßen Verbindungen sind neu und können auf folgenden Wegen hergestellt werden:

1. Ein Anilin der allgemeinen Formel II, in welcher R die oben angegebene Bedeutung besitzt, wird nach bekannten Methoden mit Hilfe von Phosgen in das entsprechende Arylisocyanat der allgemeinen Formel III umgewandelt, welches in Gegenwart oder Abwesenheit eines Lösungs- oder Verdünnungsmittels mit Hydroxylamin oder einem Salz desselben umgesetzt wird, wobei im Falle der Verwendung eines Salzes noch eine organische oder anorganische Hilfsbase eingesetzt wird. Der so erhältliche N-Aryl-N'-hydroxyharnstoff der allgemeinen Formel IV wird nach bekannten Methoden in Gegenwart von organischen oder anorganischen Hilfsbasen in einem Lösungs- oder Verdünnungsmittel mit einem starken Methylierungsmittel, z.B. Dimethylsulfat, in die erfindungsgemäße Verbindung I überführt.

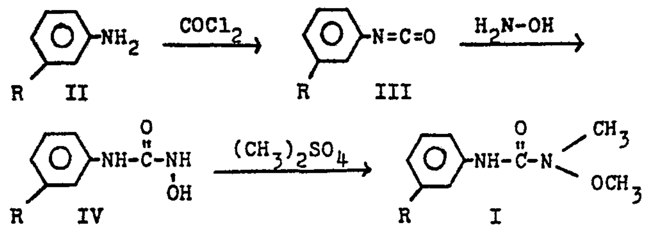

II III IV I

Anstelle des Hydroxylamins oder seines Salzes kann man auch N-Methylhydroxylamin oder O,N-Dimethylhydroxylamin oder deren Salze einsetzen, wobei im Falle der Verwendung des Dimethylhydroxylamins die anschließende Methylierung natürlich unterbleibt.

2. Ein anderer Weg, zu den erfindungsgemäßen Harnstoffen der Formel I zu kommen, besteht darin, daß man ein Anilin der allgemeinen Formel II in Gegenwart eines Lösungs- oder Verdünnungsmittels mit N-Methoxy-N-methylcarbaminsäurechlorid der Formel V unter Zugabe einer organischen oder anorganischen Hilfsbase umsetzt:

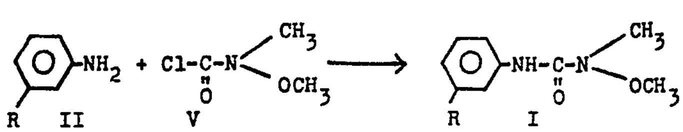
II + V I

Die zur Herstellung der erfindungsgemäßen Harnstoffe verwendeten Aniline der allgemeinen

Formel II können beispielsweise nach dem Verfahren von H. G. Gilmann et al., J.Org.Chem. *19,* 1071 (1954) hergestellt werden.

Das Verfahren 1 wird bevorzugt.

Geeignete Aniline sind beispielsweise:

m-Tertiär-butylanilin $Kp_{0,01} = 73°C$

m-Iso-propylanilin $Kp_{0,01} = 65°C$

Beispiel 1

Zu einer Lösung von 14,9 Teilen (Gewichtsteilen) metatertiär-Butylanilin und 10,1 Teilen Triäthylamin in 100 Teilen Toluol tropft man bei 20 bis 40°C 12,4 Teilen N-Methoxy-N-methylcarbaminsäurechlorid. Nach halbstündigem Rühren wäscht man die Reaktionsmischung mit Wasser, trennt die organische Phase ab und dampft ein.

Nach dem Umkristallisieren aus Methylcyclohexan erhält man 17,2 Teile N-meta-tertiär-Butylphenyl-N'-methoxy-N'-methylharnstoff vom Schmelzpunkt 82—83°C.

Beispiel 2

Zu einer gerührten Mischung aus 287 Teilen Hydroxylaminhydrochlorid, 367 Teilen Wasser und 770 Teilen Dichlormethan tropft man bei 0 bis 5°C 33 %ige Natronlauge.

Anschließend tropft man 443 Teile meta-Isopropylphenylisocyanat, gelöst in 400 Teilen Dichlormethan, zu und läßt über Nacht bei Raumtemperatur rührer. Der ausgefallene Niederschlag wird abgesaugt, mit Wasser gewaschen und getrocknet. Man erhält 505 Teile N-meta-Isopropylphenyl-N'-hydroxyharnstoff vom Schmelzpunkt 120 bis 123°C. Dieser wird in 1350 Teilen Wasser suspendiert und durch Zutropfen von 1000 Teilen einer 39 %igen Natronlauge gelöst. Unter Rühren tropft man bei 20 bis 40°C 920 Teile Dimethylsulfat zu, rührt eine Stunde bei Raumtemperatur, kühlt auf 5°C ab, saugt den Niederschlag ab, wäscht ihn mit Wasser und trocknet ihn. Man erhält 480 Teile N-meta-Isopropylphenyl-N'-methoxy-N'-methylharnstoff, der nach dem Umkristallisieren aus Methylcyclohexan bei 78 bis 79°C schmilzt.

Als Anwendungsmethoden für die neuen Wirkstoffe können das Einbringen in den Boden, die Behandlung der Bodenoberfläche oder die Behandlung aufgelaufener Pflanzen in Betracht gezogen werden. Auch Spezialanwendungen wie die Unterblattspritzung (postdirected, lay-by) kommen in Frage. Hierbei wird der Spritzstrahl so gelenkt, daß die Blätter auflaufener, empfindlicher Kulturpflanzen nach Möglichkeit nicht getroffen werden, während die Mittel auf die darunterliegende Bodenflächge oder dort wachsende unerwünschte Pflanzen gespritzt werden.

In Anbetracht der Vielseitigkeit der Applikationsmethode können die erfindungsgemäßen Mittel oder diese enthaltende Mischungen außer bei den in den Tabellen aufgeführten Nutzpflazen noch in einer weiteren großen Zahl von Kulturpflanzen zur Beseitigung unerwünschten Pflanzenwuchses eingesetzt werden. Die Aufwandmengen können dabei von 0,1 bis 15 kg/ha je nach dem Bekämpfungsobjekt schwanken.

Die Bekämpfung von gasartigen und breitblättrigen unerwünschten Pflanzen in Wintergetreide bei guter Verträglichkeit für die Kulturpflanzen wird in folgenden Beispielen demonstriert. Die Durchführung der Versuche erfolgte im Gewächshaus und im Freiland.

1. Gewächshausversuche

Als Kulturgefäße dienten Plastikblumentöpfe mit 300 $cm^3$ Inhalt, gefüllt mit lehmigen Sand mit etwa 1,5% Humus. Die Samen der Testpflanzen entsprechend Tabelle 1 wurden nach Arten getrennt flach eingesät. Unmittelbar danach erfolgte bei Vorauflaufbehandlung das Aufbringen der Wirkstoffe auf die Erdoberfläche. Sie wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen und auch gleichzeitig die chemischen Mittel zu aktivieren. Danach deckte man die Gefäße mit durchsichtigen Plastikhauben ab, bis die Pflanzen angewachsen waren. Diese Abdeckung bewirkt ein gleichmäßiges Keimen der Testpflanzen, sofern dies nicht durch die Chemikalien beeinträchtigt wurde und verhindert das Verdampfen leicht flüchtiger Substanzen.

Zum Zwecke der Nachauflaufbehandlung zog man die Pflanzen je nach Wuchsform in den Versuchgefäßen erst bis zu einer Höhe von 3 bis 10 cm und behandelte sie dann. Eine Abdeckung nach der Behandlung unterblieb. Die Aufstellung der Töpfe erfolgte im Gewächshaus. Die Versuchsperiode erstreckte sich über 4 bis 6 Wochen. Während dieser Zeit wurden die Pflanzen gepflegt und ihre Reaktion auf die einzelnen Behandlungen ausgewertet. Die nachfolgenden Tabellen enthalten die Prüfsubstanzen, die jeweiligen Dosierungen in kg/ha Aktivsubstanz und die Testpflanzen. Bewertet wurde nach einer Skala von 0 bis 100. Dabei bedeutet 0 keine Schädigung oder normaler Ablauf und 100 kein Aufgang der Pflanzen bzw. völlige Zerstörung zumindest der oberirdischen Sproßteile.

2. Freilandversuche

Es handelt sich um Kleinparzellenversuche mit lehmigem Sand und Lehm mit pH 5 bis 6 und 1 bis 1,5% Humusgehalt. Es werden Vorauflaufbehandlungen beschrieben, welche jeweils unmittelbar bis spätestens 3 Tage nach der Saat der Kulturpflanzen erfolgten. Bei den Nachauflaufbehandlungen hatten die Pflanzen Keimblätter bis mehrere echte Blätter. Das Getriede oder die unerwünschten Gräser hatten jedoch das Bestockungsstadium noch nicht überschritten. Die Unkrautflora verschiedenster Artenzusammensetzung war natürlich vorkommend. Die Substanzen werden in Wasser als Träger- und Verteilermedium emulgiert oder suspendiert und mit Hilfe einer motorgetriebenen auf einen Traktor montierten Parzellenspritze ausgebracht. Natürliche Niederschläge reichten aus, um das Keimen und Wachsen der Nutzpflanzen und Unkräuter zu gewährleisten. Alle Versuche liefen über mehrere Monate. In diesem Zeitraum wurde in gewissen Abständen die Bewertung nach der Skala von 0 bis 100 vorgenommen.

Ergebnis

Die Tabellen 2 bis 8 enthalten die Zahlenwerte der Ergebnisse: Die erfindungsgemäßen Verbindungen zeichnen sich durch eine gute Aktivität gegen ein breites Spektrum unerwünschter Pflanzen aus. Die Verträglichkeit für Wintergetreide ist ebenfalls gut. Gegenüber den bekannten Vergleichssubstanzen heben sich die erfindungsgemäßen Verbindungen durch eine deutlich bessere Bekämpfung breitblättriger Pflanzen ab. Dabei handelt es sich in erster Linie um Arten, welche bislang von in Getreide selektiven Harnstoffderivaten schwierig zu erfassen waren. Ein zweiter Vorteil der neuen Verbindungen ist ihre geringere Phytotoxizität im Vergleich zu bekannten Wirkstoffen.

Tabelle 1 — Liste der Testpflanzen

| Botanischer Name | Abkürzung in Tabellen | Deutsche Bezeichnung | Englische Bezeichnung |
|---|---|---|---|
| Alopecurus myosuroides | Alopec. myos. | Ackerfuchsschwanz | slender foxtail |
| Anthemis/Matricaria | Anthem./Matric. | Kamille | chamomile |
| Apera spica venti | Apera spica v. | Windhalm | silky bentgrass |
| Chenopodium album | Chenop. album | Weißer Gänsefuß | lambsquarters |
| Echinochloa crus galli | Echin. c.g. | Hühnerhirse | barnyardgrass |
| Galinsoga parviflora | Galins. parv. | Kleinblütiges Franzosenkraut | galant soldier |
| Galium aparine | Galium apar. | Klettenlabkraut | catchweed bedstraw |
| Hordeum vulgare | Hordeum vulg. | Gerste | barley |
| Lamium spp. | | Taubnesselarten | dead-nettle |
| Papaver spp. | | Mohn | poppy |
| Polygonum persicaria | Polyg. pers. | Flohknöterich | ladystumb |
| Stellaria media | | Vogelsternmiere | chickweed |
| Triticum aestivum | Tritic. aest. | Weizen | wheat |
| Veronica spp. | | Ehrenpreisarten | speedwell |
| Viola arvensis | Viola arv. | Ackerstiefmütterchen | wild pansy |

Tabelle 2

Vergleich der Wirkung verschiedener Phenylharnstoffe gegen einige unerwünschte Ackerunkräuter bei Vorauflaufanwendung im Freiland.

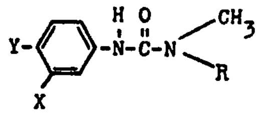

| Substitutionen R | X | Y | kg/ha | Chenop. album | Echinoc. c.g. | Galins. parv. | Polyg. pers. |
|---|---|---|---|---|---|---|---|
| | | | | Testpflanzen und % Schädigung | | | |
| $OCH_3$ | $C_4H_9$tert. | H | 1,0 | 98 | 78 | 85 | 30 |
| | | | 2,0 | 98 | 90 | 95 | 100 |
| | | | 4,0 | 100 | 96 | 100 | 100 |
| $OCH_3$ | $C_3H_7$i | H | 1,0 | 92 | 85 | 95 | 100 |
| | | | 2,0 | 100 | 95 | 100 | 100 |
| | | | 4,0 | 100 | 100 | 100 | 100 |
| $OCH_3$ | $CH_3$ | H | 1,0 | 42 | 19 | 30 | 30 |
| | | | 2,0 | 72 | 35 | 50 | 70 |
| bekannt | | | 4,0 | 98 | 50 | 85 | 100 |

0 = keine Schädigung, 100 = totale Schädigung

Tabelle 3
Wirkung von substituierten Phenylharnstoffen gegen einige unerwünschte Pflanzen bei Nachauflaufanwendung im Freiland unter Netzmittelzusatz *.

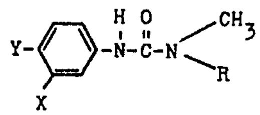

| | Substitutionen R | X | Y | kg/ha | % Schädigung Alopecurus myosuroides | Galium aparine | Lamium spp. |
|---|---|---|---|---|---|---|---|
| | $CH_3$ | H | $C_3H_7i$ | 1,0 | 95 | 8 | 50 |
| bekannt | | | | 2,0 | 100 | 12 | 70 |
| | $OCH_3$ | H | $C_3H_7i$ | 1,0 | 90 | 15 | 45 |
| bekannt | | | | 2,0 | 95 | 40 | 75 |
| | $OCH_3$ | $CH_3$ | H | 1,0 | 72 | 22 | 40 |
| bekannt | | | | 2,0 | 85 | 48 | 80 |
| | $OCH_3$ | $C_4H_9$tert. | H | 1,0 | 80 | 60 | 60 |
| | | | | 2,0 | 92 | 80 | 82 |
| bekannt | Netzmittel | | | | 8 | 5 | 0 |

* Netzmittel = Anlagerungsprodukt von 6 bis 7 Mol Äthylenoxid an 1 Mol Isoctylphenol, Aufwandmenge 2 l/ha

0 = keine Schädigung, 100 = Pflanzen abgestorben

Tabelle 4
Vergleich verschiedener Harnstoffe bei Vorauflaufanwendung (VA) bei Wintergetreide im Freiland.

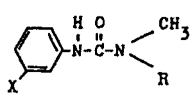

| | Substitutionen R | X | kg/ha | Hordeum vulg. | Testpflanzen und % Schädigung Alope. myos. | Anthem./ Matric. | Galium apar. | Stellaria media | Viola arv. |
|---|---|---|---|---|---|---|---|---|---|
| | $CH_3$ | $C_4H_9$tert. | 1,0 | 0 | 52 | 42 | — | 65 | 5 |
| bekannt | | | 2,0 | 2 | 55 | 79 | 2 | 85 | 10 |
| | $OCH_3$ | $C_4H_9$tert. | 1,0 | 5 | 72 | 75 | — | 92 | 45 |
| | | | 2,0 | 5 | 88 | 89 | 52 | 99 | 70 |

0 = keine Schädigung

100 = totale Schädigung

Tabelle 5
Bekämpfung unerwünschter Pflanzen in Getreidekulturen bei Vorauflaufanwendung verschiedener Phenylharnstoffe im Freiland.

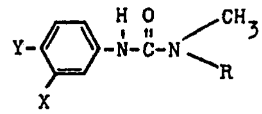

| | Substitutionen R | X | Y | kg/ha | Hordeum* vulg. | Tritic.* aestiv. | alopec. myos. | Anthem./ Matric. | Stellaria media. | Viola arv. |
|---|---|---|---|---|---|---|---|---|---|---|
| | $CH_3$ | H | $C_3H_7i$ | 2,0 | 5 | 2 | 90 | 100 | 95 | — |
| bekannt | | | | 4,0 | 28 | 14 | 99 | 100 | 100 | — |
| | $OCH_3$ | $CH_3$ | H | 2,0 | 32 | 15 | 82 | 99 | 100 | 40 |
| bekannt | | | | 4,0 | 75 | 32 | 94 | 100 | 100 | 60 |
| | $OCH_3$ | $C_4H_9$tert. | H | 2,0 | 5 | 0 | 88 | 90 | 100 | 42 |
| | | | | 4,0 | 18 | 4 | 98 | 100 | 100 | 70 |
| | $OCH_3$ | $C_3H_7i$ | H | 2,0 | 18 | 0 | 84 | 98 | 100 | 45 |
| | | | | 4,0 | 58 | 6 | 96 | 99 | 100 | 60 |

(Testpflanzen und % Schädigung: Hordeum* vulg. bis Viola arv.)

* Wintergetreide

0 = keine Schädigung, 100 = Pflanzen nicht aufgelaufen oder abgestorben

Tabelle 6

Bekämpfung wichtiger Unkräuter in Wintergetreide mit Phenylharnstoffen bei Nachauflaufanwendung im Freiland.

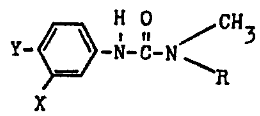

| | Substitutionen R | X | Y | kg/ha | Hordeum* vulg. | Tritic.* aestiv. | Anthem/ Matric. | Galium apar. | Stellaria media | Viola arv. |
|---|---|---|---|---|---|---|---|---|---|---|
| | | | | | Testpflanzen und % Schädigung | | | | | |
| | $CH_3$ | H | $C_3H_7i$ | 2,0 | 16 | 5 | 98 | 5 | 92 | 51 |
| bekannt | | | | 4,0 | 38 | 20 | 100 | 14 | 95 | 69 |
| | $OCH_3$ | $CH_3$ | H | 2,0 | 41 | — | 99 | 52 | 99 | 85 |
| bekannt | | | | 4,0 | 78 | — | 100 | 75 | 100 | 96 |
| | $OCH_3$ | $C_4H_9$tert. | H | 2,0 | 10 | 1 | 90 | 49 | 97 | 59 |
| | | | | 4,0 | 27 | 7 | 98 | 68 | 99 | 76 |
| | $OCH_3$ | $C_3H_7i$ | H | 2,0 | 16 | — | 100 | 32 | 100 | 52 |
| | | | | 4,0 | 64 | 5 | — | 50 | — | 80 |

* Wintergetreide

0 = keine Schädigung, 100 = Pflanzen zerstört

Tabelle 7

Verträglichkeit verscheidener Weizen-Sorten gegenüber Phenylharnstoffen bei Nachauflaufanwendung im Gewächshaus.

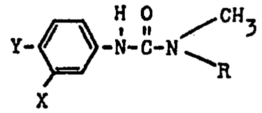

| | Substitutionen | | | | Weizen-Sorte und % Schädigung | | | | |
|---|---|---|---|---|---|---|---|---|---|
| | R | X | Y | kg/ha | Komoran | Pantus | Jubilar | Florian | Kranich |
| | $OCH_3$ | $C_4H_9$tert. | H | 0,5 | 0 | 0 | 0 | 0 | 0 |
| | | | | 1,0 | 10 | 0 | 0 | 0 | 0 |
| | | | | 2,0 | 25 | 0 | 0 | 0 | 12 |
| | $CH_3$ | $C_4H_9$tert. | H | 0,5 | 8 | 25 | 10 | 12 | 25 |
| | | | | 1,0 | 28 | 42 | 18 | 18 | 35 |
| bekannt | | | | 2,0 | 48 | 45 | 22 | 50 | 58 |
| | $OCH_3$ | $CH_3$ | H | 0,5 | 0 | 0 | 0 | 0 | 0 |
| | | | | 1,0 | 0 | 20 | 0 | 0 | 22 |
| bekannt | | | | 2,0 | 0 | 35 | 5 | 18 | 45 |

0 = keine Schädigung

100 = totale Schädigung

Tabelle 8

Bekämpfung von grasartigen und hartnäckigen breitblättrigen Unkräutern mit Phenylharnstoffen bei Nachauflaufanwendung im Freiland

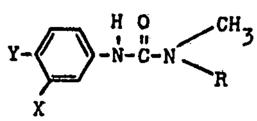

| | Substitutionen | | | | Testpflanzen und % Schädigung | | | | | |
|---|---|---|---|---|---|---|---|---|---|---|
| | R | X | Y | kg/ha | Tritic.* aest. | Alopec. myosur. | Apera spica v. | Lamium spp. | Papaver spp. | Veronica spp. |
| bekannt | $CH_3$ | H | $C_3H_7$i | 1,0 | 0 | 85 | — | 0 | 0 | 36 |
| | | | | 1,8 | 0 | 88 | 89 | 32 | 82 | 56 |
| | $OCH_3$ | $C_4H_9$tert. | H | 1,0 | 0 | 88 | 87 | 57 | 87 | 76 |
| | | | | 1,8 | 2 | 90 | 98 | 87 | 98 | 90 |

* Winterweizen

0 = keine Schädigung

100 = Pflanzen abgestorben

Die neuen erfindungsgmäßen herbiziden substituierten Harnstoffderivate können mit zahlreichen Vertreten anderer herbizider oder wachstumsregulierender Wirkstoffgruppen gemischt und gemeinsam ausgebracht werden. Solche Kombinationen dienen zur Verbreiterung des Wirkungsspektrums und erzielen zuweilen synergistische Effekte.

Außerdem ist es nützlich, die neuen erfindungsgemäßen Verbindungen allein oder in Kombination mit anderen Herbiziden auch noch mit weiteren Pflanzenschutzmitteln gemischt gemeinsam ausbringen zu können. Hierbei sind Mittel zur Bekämpfung von Schädlingen, phytopathogen Pilzen oder Wachstumsregulatoren zu nennen. Von Interesse ist ferner die Mischbarkeit mit Mineraldüngerlösungen, welche zur Behebung von Ernährungs- oder Spurenelementmängel eingesetzt werden.

Die Anwendung erfolgt, z.B. in Form von direkt versprühbaren Lösungen, Pulvern, Suspensionen auch hochprozentiger wäßrige, ölige oder sonstige Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwekken; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw. sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Äthanol, Propanol, Butanol, Chloroform, Tetrachlorkohlenstoff, Cyclohexanol, Cyclohexanon, Chlorbenzol, Isophoron usw., stark polare Lösungmittel, z.B. Dimethylformamid, Dimethylsulfoxid, N-Methylpyrrolidon, Wasser usw. in Betracht.

Wäßrige Anwendungsformen können aus Emulsionskonzentrationen, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl der Lösungsmittel gelöst, mittels Netz-, Haft-, Dispergier- oder Emulgiermittel in Wasser homogenisiert werden. Es können aber auch aus wirksamer Substanz Netz-, Haft-, Dispergieroder Emulgiermittel oder Öl bestehende Konzentrate hergestellt werden, die zur Verdünnung mit Wasser geeignet sind.

An oberflächenaktiven Stoffen sind zu nennen:

Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdatkalisalze der Dibutylnaphthalinsulfonsäure, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd Polyoxyäthylenoctylphenoläther, äthoxyliertes Isooctylphenol, Octylphenol, Nonylphenol, Alkylphenolpolyglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholpolyglykolätheracetal, Sorbitester, Lignin, Sulfitablaugen und Methylcellulose.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergesteblt werden. Feste Trägerstoffe sind z.B. Mineralerden wie Silicagel, Kieselsäuren, Kieselgele, Silikate, Talkum, Kaolin, Attapulgit, Kalkstein, Kalk, Kreide, Bolus, Löß, Ton, Dolomit, Diatomeenerde, Kalcium- und Magnesiumsulfat, Magnesiumoxid, gemahlene Kunststoffe, Düngemittel wie z.B. Ammoniumsulfat, Ammoniumphosphat, Ammoniumnitrat, Harnstoffe und pflanzliche Produkte wie Getreidemehle, Baumrinden-, Holz- und Nußschalenmehl, Cellulosepulver und andere feste Trägerstoffe.

Die Formulierungen enthalten zwischen 0, 1 und 95 Gew.-% Wirkstoffe, vorzugsweise zwischen 0,5 und 90 Gew.-%.

Zu den Mischungen oder Einzelwirkstoffen können Öle verschiedenen Typs, Netz- oder Haftmittel, Nematozide, Bakterizide, Antischaummittel (z.B. Silikone), zugesetzt werden. Die Zumischung dieser Mittel zu den erfindungsgemäßen Herbiziden kann im Gewichtsverhältnis 1:10 bis 10:1 erfolgen. Das gleiche gilt für Öle, Netz- oder Haftmittel, Fungizide, Nematozide, Insektizide, Bakterizide und Wachstumsregulatoren.

Beispiel 3

Man vermischt 90 Gewichtsteile der Verbindung aus Beispiel 1 mit 10 Gewichtsteilen N-Methyl-$\alpha$-pyrrolidon und erhält eine Lösung, die zur Anwendung in Form kleinster Tropfen geeignet ist.

Beispiel 4

20 Gewichtsteile der Verbindung gemäß Beispiel 2 werden in einer Mischung gelöst, die aus 80 Gewichtsteilen Xylol, 10 Gewichtsteilen des Anlagerungsproduktes von 8 bis 10 Mol Äthylenoxid an 1 Mol Ölsäure-N-mono-äthanolamid, 5 Gewichtsteilen Kalciumsalz der Dodecylbenzosulfonsäure und 5

Gewichtsteilen des Anlagerungsproduktes von 40 Mol Äthylenoxid an 1 Mol Ricinusöl besteht. Durch Ausgießen und feines Verteilen der Lösung in 100 000 Gewichtsteilen Wasser erhält man eine wäßrige Dispersion, die 0, 02 Gew.-% des Wirkstoffs enthält.

Beispiel 5

20 Gewichtsteile der Verbindung gemäß Beispiel 2 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Äthylenoxid an 1 Mol Isooctylphenol und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 6

20 Gewichtsteile der Verbindung gemäß Beispiel 2 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Äthylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

Beispiel 7

20 Gewichtsteile des Wirkstoffs gemäß Beispiel 2 werden mit 3 Gewichtsteilen des Natriumsalzes der Diisobutylnaphthalin-$\alpha$-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.-% des Wirkstoffs enthält.

Beispiel 8

3 Gewichtsteile der Verbindung gemäß Beispiel 1 werden mit 97 Gewichtsteilen feinteiligen Kaolin innig vermischt. Man erhält auf diese Weise ein Stäumemittel, das 3 Gew.-% des Wirkstoffs enthält.

Beispiel 9

30 Gewichtsteile der Verbindung gemäß Beispiel 2 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

Beispiel 10

40 Gewichtsteile des Wirkstoffs gemäß Beispiel 1 werden mit 10 Teilen Natriumsalz eines Phenolsulfonsäure-Harnstoff-Formaldehyd-Kondensates, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.-% Wirkstoff enthält.

Beispiel 11

20 Teile des Wirkstoffs gemäß Beispiel 2 werden mit 2 Teilen Kalciumsalz der Dodecylbenzolsulfonsäure, 8 Teilen Fettalkoholpolyglykoläther, 2 Teilen Natriumsalz eines Phenol-Harnstoff-Formaldehyd-Kondensates und 68 Teilen eines paraffinischen Mineralöls innig vermischt. Mann erhält eine stabile ölige Dispersion.

**Patentansprüche**

1. N-Phenyl-N'-methyl-N'-methoxyharnstoffderivative der Formel

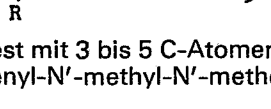,

in welcher R einen verzweigten Alkylrest mit 3 bis 5 C-Atomen bedeutet.

2. Herbizid, enthaltend ein N-Phenyl-N'-methyl-N'-methoxy-harnstoffderivat der Formel

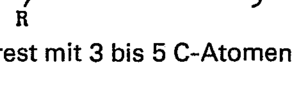,

in welcher R einen verzweigten Alkylrest mit 3 bis 5 C-Atomen bedeutet.

3. Verfahren zur Bekämpfung unerwünschten Pflanzenwuchses, *dadurch gekennzeichnet*, daß man die Pflanzen oder den Boden behandelt mit einem N-Phenyl-N'-methyl-N'-methoxyharnstoffderivat der Formel

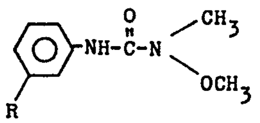

,

in welcher R einen verzweigten Alkylrest mit 3 bis 5 C-Atomen bedeutet.

4. Herbizides Mittel, enthaltend ein N-Phenyl-N'-methyl-N'-methoxy-harnstoffderivat, ausgewählt aus der Gruppe bestehend aus N-(3-tert.-Butylphenyl)-N'-methoxy-N'-methylharnstoff, N-(3-Isopropylphenyl)-N'-methoxy-N'-methylharnstoff.

5. N-(3-tert.-Butylphenyl)-N'-methoxy-N'-methylharnstoff.

6. N-(3-Isopropylphenyl)-N'-methoxy-N'-methylharnstoff.

**Claims**

1. N-phenyl-N'-methyl-N'-methoxyurea derivatives of the formula

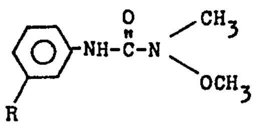

,

where R denotes a branched alkyl of from 3 to 5 carbon atoms.

2. A herbicide containing an N-phenyl-N'-methyl-N'-methoxyurea derivative of the formula

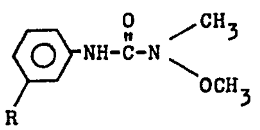

,

where R denotes a branched alkyl of from 3 to 5 carbon atoms.

3. A process for controlling the growth of unwanted plants, wherein the plants or the soil are treated with an N-phenyl-N'-methoxyurea derivative of the formula

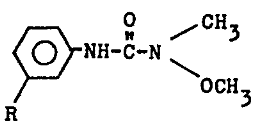

,

where R denotes a branched alkyl of from 3 to 5 carbon atoms.

4. A herbicide containing an N-phenyl-N'-methyl-N'-methoxyurea derivative selected from the group consisting of N-(3-tert-butylphenyl)-N'-methoxy-N'-methylurea and N-(3-isopropylphenyl)-N'-methoxy-N'-methylurea.

5. N-(3-tert-butylphenyl)-N'-methoxy-N'-methylurea.

6. N-(3-isopropylphenyl)-N'-methoxy-N'-methylurea.

**Revendications**

1. Dérivés de N-phényl-N'-méthyl-N'-méthoxyurée de la formule

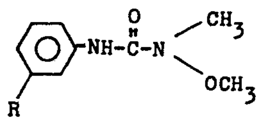

,

où R désigne un reset alkyle ramifié en $C_3$ à $C_5$.

2. Herbicide contenant un composé selon la revendication 1.

3. Procédé pour lutter contre la croissance des plantes adventices, caractérisé par le fait qu'on traite les plantes ou le sol avec un composé selon la revendication 1.

4. Agent herbicide contenant un composé choisi parmi la N-(3-tert-butylphényl)-N'-méthoxy-N'-méthylurée et la N-(3-isopropylphényl)-N'-méthoxy-N'-méthylurée.

5. N-(3-tert-butylphényl)-N'-méthoxy-N'-méthylurée.

6. N-(3-isopropylphényl)-N'-méthoxy-N'-méthylurée.